Europäisches Patentamt

**European Patent Office**

**Office européen des brevets**

(11) Publication number: **0 441 406 A1**

# EUROPEAN PATENT APPLICATION

(21) Application number: **91101811.7**

(22) Date of filing: **08.02.91**

(51) Int. Cl.⁵: **A61F 6/04**

(30) Priority: **08.02.90 US 477352**
**30.01.91 US 647662**

(43) Date of publication of application:
**14.08.91 Bulletin 91/33**

(84) Designated Contracting States:
**DE FR GB IT**

(71) Applicant: **LONDON INTERNATIONAL U.S.**
**HOLDINGS INC.**
**Route 46 West**
**Little Falls, New Jersey(US)**

(72) Inventor: **White, Nicholas David**
**42 Portland Rd.**
**Bishops Stortford, Herts(GB)**

(74) Representative: **Patentanwälte Grünecker,**
**Kinkeldey, Stockmair & Partner**
**Maximilianstrasse 58**
**W-8000 München 22(DE)**

(54) **Condom composition.**

㊼ Disclosed is a condom including a natural membrane sheath coated with a continuous barrier coating. Also disclosed is a method of coating the condom.

EP 0 441 406 A1

## CONDOM COMPOSITION

Field of the Invention

The present invention relates to an improved prophylactic (condom) construction which incorporates a barrier coating on the membrane of which the condom is formed. The barrier reduces the permeability of the condom to disease vectors.

Background of the Invention

This application is a continuation-in-part of U.S. patent application serial no. 477,352 filed February 8, 1990.

Male prophylactic devices or condoms are generally in the form of a sheath and are constructed of natural rubber or latex, or tissue membrane derived from animal sources. One type of condom in the latter category is constructed from processed lamb intestine.

Condoms constructed of latex or natural rubber are highly impermeable and serve as an effective barrier against impregnation and the organisms that carry sexually transmitted diseases (STDs), including by way of example, Herpes Simplex Virus (HSV), Human Immunodeficiency Virus (HIV) and gonorrhea. Condoms constructed from processed lamb intestine (hereafter referred to as "natural membrane" condoms), although effective against pregnancy and transmission of most STD vectors, are more permeable than rubber-based condoms.

The increased permeability of natural membrane condoms results from the construction of the membrane which is a biological material. The processed membrane includes collagen, a fibrous protein, as a major component. When examined under high magnification, the processed membrane appears to be a mat of closely spaced fibers. The membrane does not resemble a rubber based condom, which even under high magnification appears to be a continuous film.

Despite the increased permeability of natural membrane condoms as contrasted with rubber based condoms, natural membrane condoms have other characteristics which make them preferable to those made of rubber. Natural membrane condoms have a high breaking strength, yet they are reported to provide a more "natural" feel and greater sensation than rubber based condoms.

Natural membrane condoms can be constructed from several types of animal membranes, particularly those of ruminants. One type of natural membrane condom sold under the trademark FOUREX (manufactured by Schmid Laboratories, Inc., Little Falls, New Jersey) is constructed from the ceacum (or "cecum") of the lamb and has physical properties that are generally representative of natural membrane condoms. Lamb is chosen primarily for its size, availability and economy.

The first step in processing the lamb membrane is defatting the ceacum with diluted caustic soda washes. The washes also remove blood, water and other materials from the ceacum. The membrane is dried and shipped to the condom production site. Here, the membrane is rehydrated, beaded, lubricated and packed.

Sexually transmitted disease (STD) vectors are of varying size. The human AIDS (HIV) virus for example, has a particle size of 90 to 130 nm (nanometer) (average diameter) and the hepatitis B virus has a particle size of 30 to 40 nm. The natural membrane condom (e.g. FOUREX brand) is permeable to particles having an average diameter of less than 17 nm. However, the membrane used to form natural membrane condoms is generally considered impermeable to particles having an average diameter greater than about 80nm. There is a risk however, that particles having an average disameter of less than about 80 nm may pass through the membrane. Thus, although the human AIDS virus is too large to pass through the FOUREX membrane, there is some chance that the much smaller hepatitis B virus might be able to do so.

It would be desirable, therefore, to construct natural membrane condoms that are impermeable to small size STD vectors (having an average diameter of less than about 80 nm), while maintaining the other desirable characteristics of natural membrane condoms.

Object of the Invention

It is an object of the present invention to provide a natural membrane condom which is impermeable to small size STD vectors (having an average diameter of less than about 80 nm), has a high breaking strength, and provides a more natural feel than rubber based condoms.

## Summary of the Invention

The present invention relates to a condom including a natural membrane sheath coated on at least one surface with a continuous barrier coating. The invention also relates to a method of applying the coating to a surface of the condom.

## Detailed Description of the Invention

The object of the invention is met by providing a natural membrane condom having a thin, polymeric, impermeable, flexible and continuous barrier coating capable of adhering to the natural membrane forming the condom.

The natural membrane used to construct condoms is obtained from dried lamb intestines and has a variable fat content. Various materials were evaluated as suitable polymeric coatings for natural membrane condoms. The materials evaluated included: natural rubber latex, an acrylic copolymer, polyvinylalcohol, a polyurethane emulsion, a plasticized nitrocellulose composition, a silicone wax and a silicone coating. It has now been unexpectedly discovered that the plasticized nitrocellulose composition provided a thin, continuous film which adhered well to the dry natural membrane. The natural rubber latex and the polyurethane emulsion also formed thin continuous films. Although the natural rubber latex and polyurethane emulsion films adhered to the surface of the dry natural membrane, they delaminated upon hydration of the coated condom. On the other hand, the silicone materials adhered well to the natural membranes, but a continuous barrier film could not be formed.

The coating for the natural membrane condoms of the present invention is a nitrocellulose material. However, plasticized nitrocellulose is especially preferred. The plasticized nitrocellulose composition described above was prepared from a clear lacquer adhesive. One preferred plasticized nitrocellulose composition is set forth in Table 1 below.

### TABLE 1

| | |
|---|---|
| 6.8%[1] | 70% SS grade[2] 5/6 second[3] nitrocellulose |
| and | 30% isopropanol |
| 6.8% | 70% SS grade 40/60 second nitrocellulose[4] |
| and | 30% isopropanol |
| 58% | 200 proof ethanol |
| 13.9% | 99% ethylacetate |
| 3% | butylacetate |
| 11.5% | Santisizor 160 (butylbenzylphthalate) |

(1) Percentages are given by weight.

(2) "SS grade" means solvent soluble due to low (10.9% to 11.3%) nitrogen content.

(3) "5/6 second" refers to the viscosity range, which is a function of the degree of nitration of the nitrocellulose and is measured in seconds. Viscosity is measured by the falling ball method disclosed in ASTM D301-72 and ASTM D1347-74.

(4) "40/60 second" refers to the viscosity range, as above.

It should be obvious to one skilled in the art that the invention may be practiced using the same nitrocellulose with other plasticizers or other nitrocellulose with the above-described or other plasticizers.

Nitrocellulose is commercially available and can be purchased from Hercules, Inc., Scholle Corp. and Wolff Cellulosics/Mobay Corp. The nitrocellulose composition described above is preferred due to its strong adhesive property and its flexibility when dried. To prepare a coating solution, the nitrocellulose is dissolved in an organic solvent. Any organic solvent may be used provided it is compatible with the plasticized nitrocellulose composition. Among the preferred solvents for use in the invention are acetone, methanol, propanol, ethanol, and other lower alcohols. The coating solution can comprise from about 30% to about 70% by weight of the plasticized nitrocellulose composition with the balance being an organic solvent at 15 to 40°C. The preferred coating solution includes between about 50% and 70% by weight of plasticized nitrocellulose composition and between 30% and about 50% by weight of organic solvent. One particularly preferred coating solution includes 60% by weight of the plasticized nitrocellulose composition described in Table 1 in acetone at 20 to 25°C.

The coating can be applied to the dry condom in any one of several ways. The coating solution can be sprayed or brushed onto the condom. Alternatively, the coating can be applied by dipping the condom in the coating solution. The coating may be applied to either the inner or outer surface of the condom, or both, but is applied preferably to only one surface of the condom. The coating may be applied to one surface and then the condom may be reversed. For example, the outside surface may be coated and the condom may then be inverted "inside-out" so that the coating is on the inside surface of the final condom product.

To coat the outer surface of the condom, for example, the dry natural membrane condom is inflated prior to being immersed in the coating solution. The dry natural membrane condom is gently inflated in an enclosed space which is open to the atmosphere (an "open system") with compressed air at a flow rate of 40 to 60 cm$^3$/second and at a preferred rate of 50 cm$^3$/second. The air flow rate entering the condom is adjusted to ensure that the condom is fully inflated when dipped. Inflating the dry natural membrane condom prior to dipping ensures that no wrinkles occur in the condom during dipping. The condom can also be supported by suitable mechanical means.

The inflated condom is immersed in the coating solution for a dwell time of between about 5 and 15 seconds; the preferred dwell time is between about 8 and 12 seconds. The coated condom is then withdrawn from the coating solution at a withdrawal speed of between about 1 and 3 cm/second; the preferred withdrawal speed is between about 1.5 and 2.5 cm/second. The condom is then allowed to dry in a vertical position with the tip pointing up for between about 2 and 10 minutes; the preferred drying time is about 5 minutes at between 35° and 50°C, preferably 45°C, and at ambient relative humidity (20% to 80% R.H.). The coated condom may then be deflated and then hydrated by immersion in deionized water for between 1 and 3 hours with a preferred hydration time of about 2 hours. The coating solution is tacky when applied to the condom. After the solvents have evaporated from the coating solution applied to the condom, the resultant dry film has very low tack. Thereafter the condom may be lubricated using any conventional condom lubricant (e.g. nonoxynol) and packed.

Example 1 - Coating the Condom

To coat the natural membrane condom, a nitrocellulose solution containing 60% by weight of the plasticized nitrocellulose composition described in Table 1 and 40% by weight acetone at 20°C was loaded in a tall glass container. A dry natural membrane condom was gently inflated in an open system with compressed air at a flow rate of 50 cm$^3$/second. Inflating the dry condom prior to dipping ensures that no wrinkles appear on dipping. The inflated condom was immersed for 10 seconds in the nitrocellulose coating solution. The condom was then withdrawn from the solution at a withdrawal speed of 2.0 cm/second. The coated natural membrane condom was dried with hot air at about 45°C while being maintained in a vertical position with the tip pointing up for 5 minutes. After about five minutes the dried condom was deflated and then hydrated by placing it in deionized water for 2 hours. The condom was then lubricated with a lubricant formulation comprising 74.3% deionized water, 25% propylene glycol preserved in 0.7% parabens. A 2 milliliter volume of the lubricant was injected into a conventional condom foil package made of polymer-coated aluminum foil, prior to heat sealing. The package was then heat sealed in conventional fashion.

Example 2 - Permeability of the Coated Condom

Natural membrane condoms which had been coated, lubricated and packed according to Example 1, were tested for permeability. Each condom was unpacked and unrolled, and the lubricant washed off carefully with deionized water. A plastic tube having a length of 15 cm and a diameter 3.8 cm was attached to the open end of the coated natural membrane condom using a rubber ring and metal slip. An aqueous solution of a small particle fluorescent chromophore of 17 nm particle size was used as a challenge solution to test the barrier properties of the coated condom. The chromophore solution was fluorescein isothiocyanate dextran (molecular weight 150,000, obtained from Sigma Co., St. Louis) in deionized water. The solution contained 0.1% by weight of the chromophore in de-ionized water. The condom with the attached tube was filled with this solution to a point 10 cm up the tube. This gave a positive pressure head of 1.0 kPa, across the membrane of the condom when the condom/tube arrangement was placed in deionized water, the base of the tube being level with the surface of the deionized water, such that the level of solution inside the tube was 10 cm higher than the surface of the deionized water. (The deionized water will hereafter be referred to as the "analytical solution"). 2,500 mL of analytical solution was used at ambient temperature.

The fluorescence level of the analytical solution was monitored over 4 to 6 hours using a fluorimeter. The fluorimeter used was an LS-S Luminescence Spectrometer manufactured by Perkin -Elmer. The effectiveness of the barrier provided by the coated natural membrane condom was evaluated by measuring the presence, if any, of the fluorescent chromophore, in the analytical solution. The experiment was undertaken in triplicate. An uncoated natural membrane condom was used as a positive control and a coated natural membrane condom in deionized water was used as a negative control. The results of this experiment are set forth in Table 2.

## TABLE 2

### The Barrier Properties of Coated Natural Membrane Against a Challenge Material of Particle Size 17 nm

#### Analytical Solution Fluorescence Level

| Sample | | 0 hour | 1 hour | 2 hours | 3 hours | 4 hours | 5 hours |
|---|---|---|---|---|---|---|---|
| 1 | X̄ | 2.0 | 1.9 | 2.0 | 2.3 | 2.9 | 3.4 |
|   | SD | 0.7 | 0.4 | 0.3 | 0.3 | 0.2 | 0.2 |
| 2 | X̄ | 1.7 | 1.8 | 2.1 | 2.2 | 2.5 | 2.9 |
|   | SD | 0.2 | 0.1 | 0.1 | 0.4 | 0.2 | 0.5 |
| 3 | X̄ | 1.8 | 1.9 | 2.0 | 1.9 | 2.4 | 2.6 |
|   | SD | 0.1 | 0.2 | 0.2 | 0.3 | 0.3 | 0.2 |
| 4 | X̄ | 2.0 | 4.3 | 20.2 | 17.5 | 22.9 | 43.5 |
|   | SD | 0.3 | 1.4 | 7.8 | 2.8 | 1.3 | 17.0 |
| 5 | X̄ | 1.6 | 1.3 | 1.4 | 1.5 | 1.3 | 1.3 |
|   | SD | 0.4 | 0.1 | 0.1 | 0.2 | 0.2 | 0.3 |
| 6 | X̄ | 2.2 | 1.8 | 2.2 | 1.3 | 2.0 | 1.7 |
|   | SD | 0.4 | 0.1 | 0.3 | 0.2 | 0.4 | 0.2 |

### Legend

| | | |
|---|---|---|
| 1 | – | Coated condom/Challenge solution |
| 2 | – | Coated condom/Challenge solution |
| 3 | – | Coated condom/Challenge solution |
| 4 | – | Uncoated condom/Challenge solution |
| 5 | – | Uncoated condom/De-ionized water |
| 6 | – | Coated condom/De-ionized water |
| X̄ | – | Mean of five replicates |
| SD | – | Sample standard deviation (n-1) |

The challenge material includes a fluorescent chromophore of 17 nm particle size in the analytical solution. This is smaller than the smallest known STD vector particle. The results set forth in Table 2 show that over a five hour period, the coating offers an effective barrier to the 17 nm chromophore material and therefore acts as a barrier to STD vectors.

Example 3 - Breaking Strength of the Coated Condom

The coated condom was tested according to the methods of the British standard test BS3704 (1979) for natural rubber condoms. Tensile strength, load at breakage, and elongation at breakage are measured by testing rings cut from sample condoms, measuring the average thickness and then testing on the appropriate equipment. Three samples (two rings from each condom) were tested.

Table 3 shows the peak load at break, in Newtons, of the coated condom compared with a control of a commercially available uncoated natural membrane (FOUREX) condom.

## TABLE 3

### The Peak Load at Break (N) of Coated condom

| Sample | Load at Break (N) | |
|---|---|---|
| | Mean (n=6) | Sample Standard Deviation |
| Coated natural membrane condom | 45.2 | 5.3 |
| Uncoated natural membrane condom | 26.8 | 6.0 |

The load-at-break of the coated condom was at least 35% greater than the uncoated condom. This represents a considerable improvement in the physical strength of the condom and will give the user more confidence that the membrane will not tear during normal use.

**Claims**

1. A condom comprising a natural membrane sheath bearing a barrier coating.

2. The condom of claim 1 wherein the coating is continuous.

3. The condom of claim 2 bearing said coating on at least one surface.

4. The condom of claim 3 wherein said coating is at least on the outer surface of the condom.

5. The condom of claim 3 wherein said coating is at least on the inner surface of the condom.

6. The condom of claim 1 wherein said coating comprises nitrocellulose.

7. The condom of claim 6 wherein said coating comprises plasticized nitrocellulose.

8. The condom of claim 7 wherein said plasticized nitrocellulose coating comprises an composition including nitrocellulose, ethanol, ethyl acetate, butyl acetate and butyl benzyl phthalate.

9. The condom of claim 7, wherein said coating is applied from a coating solution containing said plasticized nitrocellulose dissolved in a solvent.

10. The condom of claim 9, wherein said solvent is selected from group consisting of acetone, ethanol, methanol, propanol, lower alcohols and mixtures thereof.

11. The condom of claim 9 wherein said coating solution contains between about 30% to 70% by weight of said solution of said plasticized nitrocellulose.

12. The condom of claim 9 wherein said plasticized nitrocellulose coating solution comprises between about 50% to about 70% of said composition by weight.

13. The condom of claim 12 wherein said plasticized nitrocellulose coating comprises about 60% plasticized nitrocellulose composition by weight.

14. The condom of claim 1 wherein said coating is a barrier to particles having a diameter of at least about 17 nanometers.

15. The condom of claim 1 wherein said natural membrane is animal intestine.

16. The condom of claim 15 wherein said animal intestine is ruminant cecum.

EP 0 441 406 A1

17. The condom of claim 16 wherein said ruminant is lamb.

18. A method for coating a natural membrane condom with a plasticized nitrocellulose barrier coating, comprising:
supporting the condom;
applying said coating to the supported condom; and
drying the coated condom.

19. The method of claim 18 wherein said coating comprises a plasticized nitrocellulose composition dissolved in a solvent at a concentration between about 30% to 70% by weight of composition.

20. The method of claim 19 wherein said plasticized nitrocellulose composition comprises nitrocellulose, ethanol, ethyl acetate, butyl acetate and butyl benzyl phthalate.

21. The method of claim 18 which comprises applying said coating by brushing said coating onto the condom.

22. The method of claim 18 wherein said coating step comprises spraying said coating onto the condom.

23. The method of claim 18 wherein said coating step comprises dipping the condom into a reservoir containing said coating.

24. The method of claim 18 wherein said condom is supported by air inflation means in a wrinkle free fashion.

25. The method of claim 18 wherein said condom is supported by mechanical means in a wrinkle free fashion.

26. A method for coating a natural membrane condom with a plasticized nitrocellulose coating, comprising:
supporting a condom;
applying a coating solution including nitrocellulose, 200 proof ethanol, ethyl acetate, ethylacetate and butylbenzylphthalate, to said supported condom by dipping the condom in said coating solution for between about 8 to 12 seconds;
withdrawing said coated condom from said solution at a rate between 1 and 3 cm/second;
drying said condom for between about 2 and 10 minutes at $35^\circ$ to $50^\circ$C and at ambient relative humidity; and
hydrating the condom in deionized water for about 1 to 3 hours.

27. A condom, comprising:
a natural membrane sheath with a continuous barrier coating,
said coating is applied from a coating solution including a nitrocellulose composition dissolved in acetone, at a ratio of 60% by weight nitrocellulose composition to 40% acetone;
said composition comprising 6.8% of 70% SS 5/6 second nitrocellulose and 30% isopropanol, 6.8% of 70% SS 40/60 second nitrocellulose and 30% isopropanol, 58% of 200 proof ethanol, 13.9% of 99% ethylacetate, 3% of butylacetate, and 11.5% butylbenzylphthalate, where percentages reflect percent by weight.

8

European
Patent Office

**EUROPEAN SEARCH
REPORT**

Application Number

**EP 91 10 1811**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 904 647 (STILLMAN) <br> * Abstract, lines 1-3 * | 1-5,14-18 | A 61 F 6/04 |
| Y | | 6-13, 19-27 | |
| Y | FR-A-2 578 741 (SHISEIDO) <br> * Table 1 * | 6-13, 19-27 | |
| A | WO-A-8 907 428 (SCHMID) <br> * Page 1, lines 23-25; page 2, lines 19-20 * | 15-17,27 | |
| A | US-A-3 956 529 (ADDISON) <br> * Column 3, lines 40-45 * | 7-13,26, 27 | |
| A | US-A-4 527 988 (LUTZ) | | |

| | |
|---|---|
| | TECHNICAL FIELDS SEARCHED (Int. Cl.5) |
| | A 61 F |

The present search report has been drawn up for all claims

| Place of search | Date of completion of search | Examiner |
|---|---|---|
| The Hague | 02 May 91 | BARTON S.A. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

 

& : member of the same patent family, corresponding document